# EUROPEAN PATENT APPLICATION

(11) **EP 1 407 790 A1**
(43) Date of publication of application: **14.04.2004**
(21) Application number: 03103713.8
(22) Date of filing: 07.10.2003
(51) Int. Cl.: A61L 9/14, B65D 83/16, B05B 12/02

(54) **Improved olfactory stimulating material dispensing apparatus**

(30) Priority: 10.10.2002 MY 0223778
(71) Applicant: Spy Marketing Sdn. Bhd., Bandar Sri Damansara, Kuala Lumpur 52200 (MY)
(72) Inventor: CHON, Khor K., 52200, Kuala Lumpur, (MY)
(74) Representative: King, James Bertram

(57) **Abstract**

The present invention relates to an improved dispensing apparatus for olfactory stimulating material. Utilising a micro-controller and software control, it is user configurable to be able to operate in a variety of operating modes. Incorporating ambient condition sensor into the apparatus allows it to operate only when certain ambient conditions are met. This gives the apparatus the flexibility to be adaptable to a wide variety of operating conditions. In particular, multiple actuations per dispensing action and operation when ambient conditions change can be configured by the user.

## Description

### Field of Invention

The present invention relates to dispensers for dispensing olfactory stimulating materials for improving the surrounding air quality, and more particularly, to an improved automatic dispenser capable of being user configured to operate in one of a plurality of operating modes, including normal operation, day / night mode, state change triggered dispensing mode. Additionally, the user can set the amount of olfactory material dispensed per dispensing action.

### Background of the Invention

Dispensers that provide an olfactory stimulating material, such as an air freshener composition, are known, and often installed, among other places, in public rest rooms. Such dispensers provide to the surrounding atmosphere an olfactory stimulating material from a refill canister or other source of olfactory stimulating material. Since the need for air freshening varies widely, depending on the application, dispensers which cannot be configured with wide flexibility are either operating even when the need for freshening is not great, resulting in waste, or operating with insufficient frequency or intensity when the need is greatest, resulting in insufficient air freshening. US patent 5,105,133 attempts to overcome this problem by introducing a multiple mode dispenser. However, being controlled by logic ICs, its operation is restricted to only a few hardwired (i.e. non-configurable) choices.

New uses have been found for such dispensers. Hotels, for example, are installing such devices in rooms to welcome their guests when they enter the room for the first time. A refreshing whiff of fragrance helps weary hotel guests feel more invigorated. They are increasingly being installed in cars and other forms of transportation. Since the environment in which these dispensers are placed can vary widely, from traditional places such as rest rooms to cars and hotel rooms, the dispenser must have sufficient flexibility designed into it to handle diverse operating conditions. Among important features such dispensers must have are capability to dispense variable amount of olfactory stimulating material to suit room / compartment size and ability to sense when ambient conditions changed, such as when a guest enters the room or when the driver opens the car door or when the car is moving. Most dispensers, such as that described in US patent 5,038,972, have a light sensor to sense a change from daytime to night, but cannot detect when a person enters a room or open a door. Thus, they cannot effective dispense the olfactory stimulating material at the appropriate time. They should operate only when guests enter the room, and not continuously, so that they may be economical to operate.

In addition, sources of olfactory stimulating material are in refill canister, they have to be replaced when empty, or when near empty, i.e., when a predetermined amount of the olfactory stimulating material has been dispensed. However, in a programmable dispenser that does not operate solely on a continual basis, but rather only during set periods (such as during periods of daylight or during preset hours of operation) and/or does not operate to provide a constant level of olfactory stimulating material, but rather with variable frequency or intensity, the dispensing of a predetermined amount of olfactory stimulating material, such as when the source of olfactory stimulating material is empty or near empty, can occur at different times depending on the operation of the dispenser. Accordingly, the conventional assumption that a predetermined amount of olfactory stimulating material has been dispensed after, for example, thirty days will no longer suffice.

### Summary of the Invention

Earlier dispensing mechanisms, controlled by logic ICs are not flexible enough to meet the new operating requirements set out above. To cater for these new requirements, it is the object of this invention to introduce an improved micro-controller based dispensing mechanism, using software control that gives the mechanism unlimited flexibility to operate in a variety of modes and under a variety of operating conditions.

In particular, the user can set the amount of olfactory stimulating material to be dispensed per dispensing action, via multiple actuations of the deodorant source. In addition, the apparatus is equipped with an ambient condition sensor, which allows it to sense its surroundings. It can be configured to perform preset actions if certain ambient conditions are met, e.g. to dispense only when a guest enters a room, or to dispense only when a car engine is running.

### Brief Description of the Drawings

Fig. 1 shows the external front view of the apparatus.
Fig. 2 shows the internal top part of the apparatus.
Fig. 2a shows the internal top part of the apparatus with the front console panel removed.
Fig. 3 shows the dispenser configuration and status routine flow chart.
Fig. 4 details the dispenser Operation Routine flow chart.
Fig. 5 details the Actuation Routine flow chart.
Fig. 6 outlines settings sequence.

### Detailed Description of the Invention

### Physical Assembly

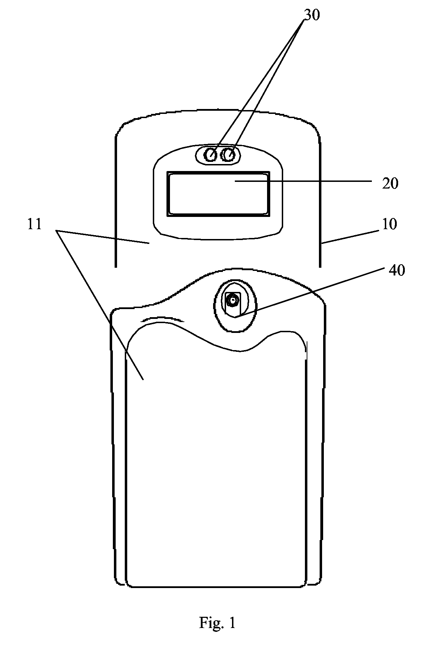

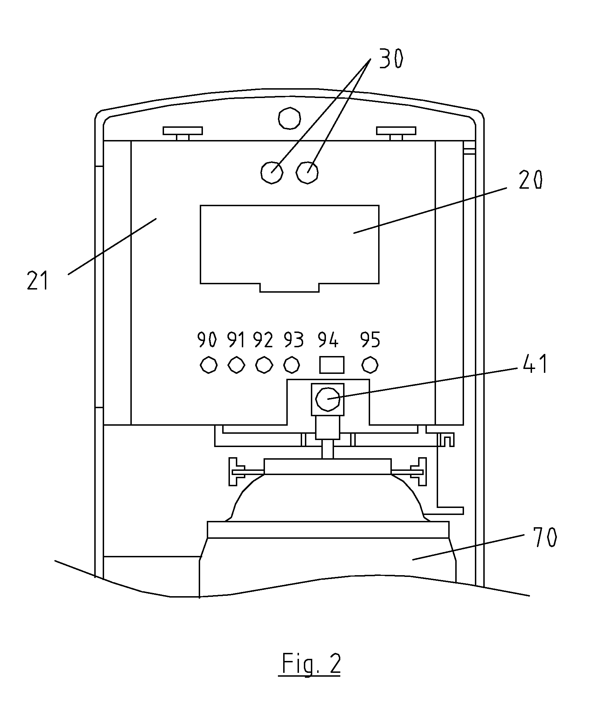

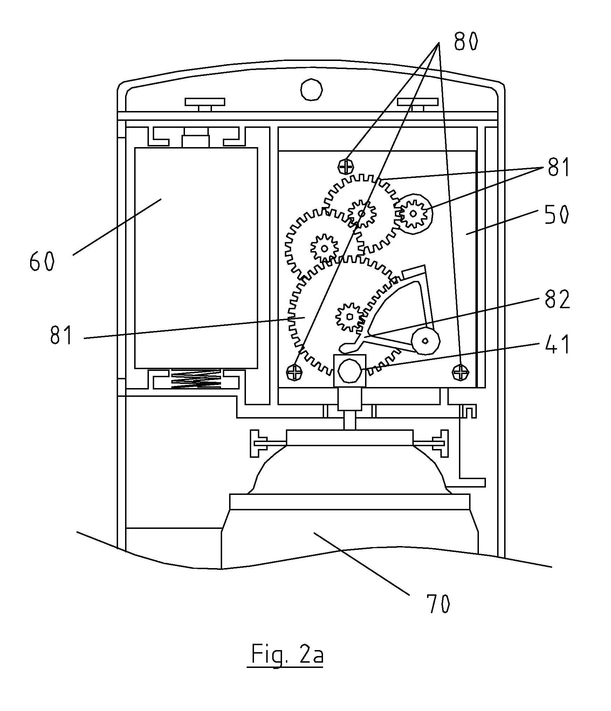

Referring to Fig. 1, a dispenser in accordance with the present invention is shown. The dispenser comprises of a housing (10) with a front cover (11), a console panel (21) (Fig 2) with LCD display (20), sensors (30), buttons and switches (90 to 95) displayed thereon, a controller board fixed immediately behind the console panel (21) and a canister-can (70) containing olfactory stimulating material. There is provided an opening (40) on the housing (10) through which the nozzle (41) of the canister-can could dispense olfactory stimulating material into the atmosphere.

Referring to Fig. 2, which shows the view when the front cover (11) of the housing (10) is opened. Mounted on the controller board (not shown) and as seen through predetermined openings and cuttings on the console panel (21) are setting buttons labeled as Set (90), Ret (91), Hr (92) & Min (93), an on/off switch (94) for selecting timer mode on or off, a button labeled as Welcome (95) for actuating sensors, sensors (30) and a LCD display (20). A controller board (not shown) is releasably fastened to the console panel (21). Referring to Fig. 2a which shows the view when the console panel (21) is removed. Within the top part of the housing are mounted main battery compartment which can accommodate a single D size battery (60) and a mounting plate (50) with a direct current (DC) electric motor (not shown) mounted at the back driving a set of gear assembly (81) mounted on the front part of the mounting plate (50). The gear assembly is operationally coupled to an actuator (82) that moves downwards each time the DC motor is excited. In normal state when the DC motor (not shown) is not excited, the actuator (82) is resting on top of the dispensing nozzle head of the canister-can (70). The mounting plate (50) is fixedly secured to the housing by releasable fasteners engaging internally threaded posts (80). A dividing wall extends from the housing between the power supply compartment and the mounting plate (50).

The components of the controller board are mounted on a circuit board. The controller includes a microcontroller unit (MCU) having a real time clock, resident memory and standby battery. The MCU is connected to a timer mode selection switch and setting buttons, a battery strength detector and optionally, either an internal or external condition sensor that is mounted on the console panel. The timer mode selection switch, welcome button and setting buttons are mounted on the controller board and are positioned facing front direction and displayed on the console panel (21) through openings on the console panels. Wire harnesses connect the power supply source to the controller board and between the DC motor and the controller board, thereby linking operational all components to the microcontroller unit (MCU) and allowing the controller to control the actuator movements and hence the frequency of opening of the valve mechanism of the nozzle to dispense a spray of material through the nozzle (41) and the length of time the valve mechanism is open for such dispensing as well as the displays on the LCD display.

Within the lower part of the housing (10) is a compartment that accommodates a canister-can (70) containing olfactory stimulating material. Each time the nozzle head of the canister-can is depressed by the actuator (82), a spray of olfactory stimulating material is dispensed to the ambient atmosphere via the opening (40). (Refer to Fig. 2a).

### Functional Description

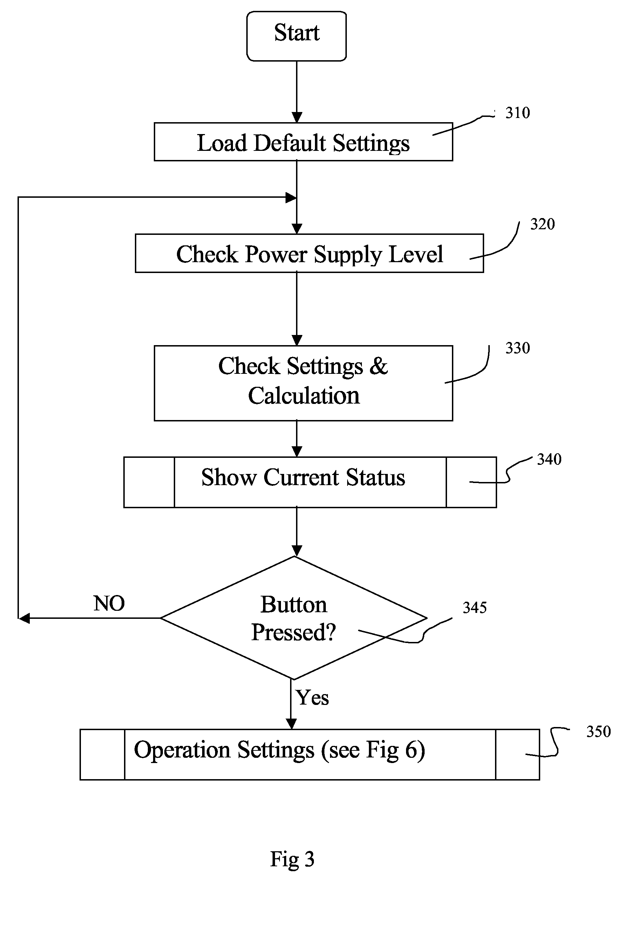

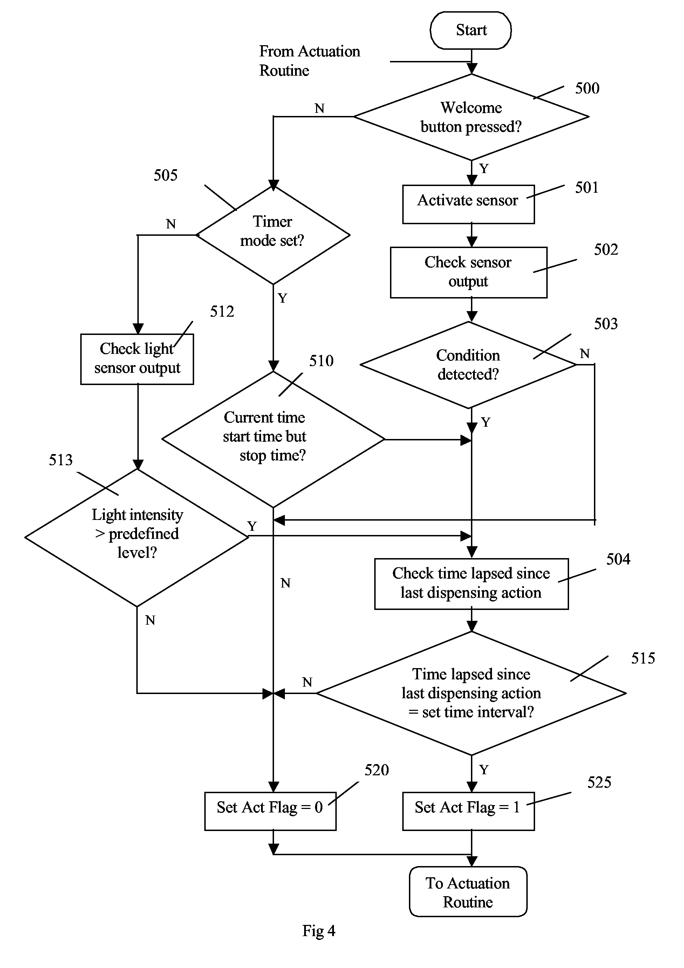

The resident memory stores programmed dispenser operation settings and a main program executed by the MCU. The main program includes a load default settings routine (310), check power supply level routine (320), check settings & calculation routine (330), display status routine (340) and operation setting routine (350) (see Fig 3). Fig 4 shows the dispenser operation routine blocks.

The microcontroller includes non-volatile memory for storing dispenser's default operation settings. The default operation settings are as follow:

| Description | Setting |
|---|---|
| 1) Real Time Clock | 12:00 am |
| 2) Weekday | Monday |
| 3) Timer | Sunday & Saturday OFF, others ON |
| 4) On Time | 8:00 am |
| 5) Off Time | 5:00 pm |
| 6) Refill Spray Count | 3000 |
| 7) Spray Interval | 30 minutes |
| 8) Actuation Count | 1 |

The MCU will check for configured settings in routine (340) before showing the status on the display.

There are four buttons i.e. "Set", "Ret", "Hr" & "Min" for settings includes a "Real Time Clock" and Weekday" setting, an "Timer" setting, a "Spray Interval" setting, a "Refill Spray Count" setting and a "Actuation Count" setting. Another button, "Welcome" is used for sensor activation. There is also provided an on/off switch for selecting timer mode on or off.

The Real Time Clock and Weekdays settings set the current time and day of the week. The Timer settings set the times for dispensing actions to begin and end for each of the weekdays when the timer is operating on "ON" mode. The Spray Interval setting sets the time interval between dispensing actions. The Actuation Count Setting sets the number of sprays per dispensing action. The number of sprays per dispensing action is generally sets between one and three but can be a higher number if necessary. If the actuation count is sets at three, the motor will be excited three times, causing the actuator to move downwards and back to its resting position three times. The Refill Spray Count setting allows the controller to calculate the amount of olfactory stimulating material remaining in the container.

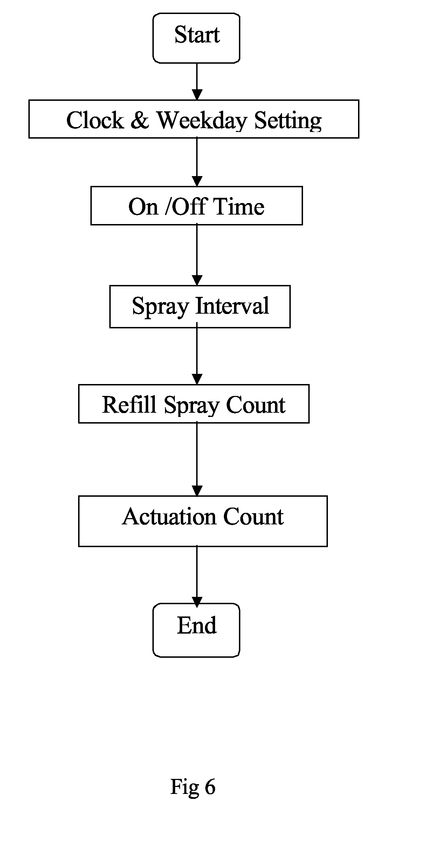

The setting procedures are now described. If the user presses the "Set" button, 345, the MCU will switch to setting mode by blinking the CLOCK symbol. Under setting mode, pressing the "Hr" or "Min" continuously will roll the setting automatically (incremental speed) and press the "Ret" at any time under setting mode will return to main screen. Under setting mode, if no buttons pressed are after 10 seconds, the system will switch to main screen automatically. Fig 6 shows the operation settings sequence.
a) Clock and Weekday Setting
   - Press the "Hr" button will change the current clock hour,
   - Press the "Min" button will change the current clock minutes,
   - When fmish, press the "Set" button once for current weekday setting,
   - Press either "Hr" or "Min" button to select current weekday,
   - When fmish, press the "Set" button once for operation days setting, the weekday symbol will blinks,
   - Press the "Hr" button to move from Sunday to Saturday and press the "Min" to change the status (ON or OFF) for the respective day
   - When finished, press the "Set" button to go to next setting, on timer
b) Timer setting
   - During on timer setting, the ON symbol will blink. Press the "Hr" button to change the hour setting and "Min" to change the minutes setting,
   - When fmish, press "Set" button once to go to Off timer setting
   - During off timer setting, the OFF symbol will blink. Press the "Hr" button to change the hour setting and "Min" to change the minutes setting,
   - When finish, press "Set" button once to go to next setting, Interval
c) Spray Interval
   - During interval setting, the Interval symbol will blink. Press the "Hr" button to increase interval and "Min" to decrease interval. The valid interval range is from 1 to 99 minutes
   - Press the "Set" button once to go to next setting, Refill Spray Count.
d) Refill Spray Count
   - During refill spray count setting, the Count will blink. Press the "Hr" button to increase upper two digits count and press the "Min" button to change the lower two digits count. The setting range are from 1 9900 counts
   - Press the "set" button once to go to next setting, Refill Spray Count.
e) Actuation Count
   - During Actuation Count setting, the current selection (H, M, L). Press either "Hr" or "Min" button to change the spray volume from high (H-three sprays) to medium (M-two sprays) or low (L-one spray),
   - Press the "Set" button once to end the setting

Under normal operation, pressing the "Ret" button will display the current spray count, the Count symbol will on. Pressing the "Hr" button during this time will reset the spray count. Press the "Ret" once again will display the number of days used, and the Life symbol will on. Press the "Ret" button one more will return to main screen. The system will return to main screen automatically 10 seconds later if no buttons is pressed.

Under normal operation, pressing the "Hr" button and hold for more than 3 seconds will toggle the real time clock display, AM/PM and 24 hours mode and the buzzer will beep once. Under 24 hour mode, no AM/PM is displayed.

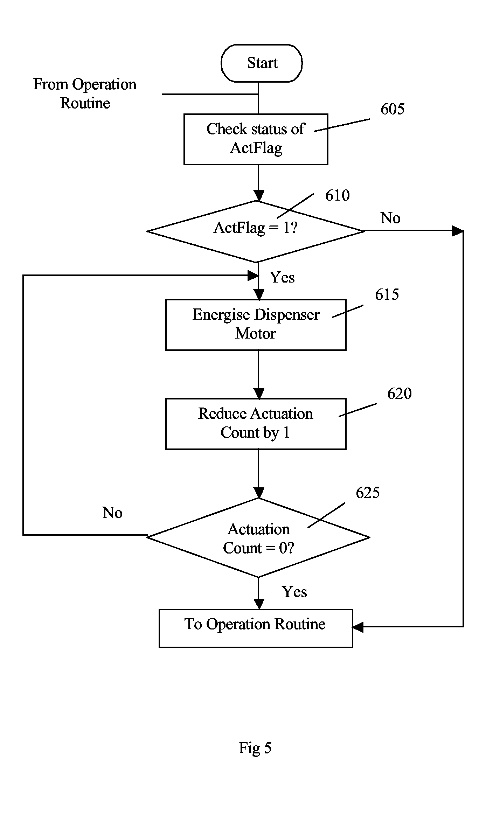

If a sensor is installed, pressing the "Welcome" button will activate the welcome spray function (buzzer will beep once). The Operation Routine in Fig. 5 begins operational ten minutes after the "Welcome" button is pressed.

The operation of the dispenser will now be described with the help of flow-charts. When the MCU executes the dispenser status routines (320-340) during initialization (when power is applied to the controller for the first time) as shown in Fig 3, the MCU loads default settings to memory and then the MCU will loop through the status routines (320-340). At first, the MCU polls the battery strength detector to determine the strength of the batteries in the power supply (320). The MCU also calculates the amount of olfactory stimulating material remaining in the container in routine (330) by subtracting the amount of olfactory stimulating material dispensed from the container (dependent on the number of sprays executed and the duration of each spray) by the amount of olfactory stimulating material originally in the container as determined by the Refill Spray Count setting. With this feature, the MCU is able to determine when the olfactory material in the canister-can is near empty and the status will be displayed in the LCD display accordingly.

In operation, the MCU executes the Operation Routine and Actuation Routine shown in Fig 4 and Fig 5 respectively.

The Operation Routine shown in Fig 4 will only be activated if the main battery is inserted. The controller is powered by a main and a stand-by battery. The MCU will first check if "Welcome" button is pressed and if it is pressed, the sensor will be activated (502) after a time lag of ten minutes. The output of the sensor will be monitored (502) to check if actuating condition has been detected (503). The actuating condition will depend on the types of sensor installed. The sensor could be a vibration sensor to detect closing of door, a proximity sensor or motion detector to detect entrance of a guest into a room, a light sensor to detect a change in light luminosity on opening of a door or temperature sensor or the like. If the actuating condition is detected, the MCU will check the time lapsed since the last dispensing action (504) and compared with the set time interval between dispensing action (515). If this time interval is reached, the actuation flag will be set (ActFlag=1) (525). If the actuating condition is not detected, the actuation activation flag will be cleared (ActFlag=0) (520).

If the "Welcome" button is not pressed, the MCU check if the timer is on (505). If so, it will check the current time and determine if the current time is between the times the dispenser is to be operational (510). If so, the MCU will check the time lapsed since the last dispensing action (504) and compared with the set time interval between dispensing action (515). If this time interval is reached, the actuation flag will be set (ActFlag=1) (525). If the timer is off, the MCU will monitor the output of a light sensor (512). If the luminosity exceeds certain predefined level, the MCU will check the time lapsed since the last dispensing action (504) and compared with the set time interval between dispensing action (515). If this time interval is reached, the actuation flag will be set (ActFlag=1) (525). If the light intensity is below predefined level, the actuation activation flag will be cleared (ActFlag=0) (520).

The Actuation Routine will now be described. The MCU will only run the actuation routine shown in Fig 5 if Operating Routine has set the ActFlag. It checks this with routine (605). If ActFlag is set, the MCU send a signal to activate the motor and gear assembly (615). An actuator operational connected to the DC motor through the gear system will move down and then back to its resting position each time the motor is excited. The actuator is resting above and on top of the head of the nozzle of a canister-can. The downward movement of the actuator opens the valve of the nozzle mechanism and thereby dispensing a spray of olfactory stimulating material into the atmosphere. The Actuation Count Setting determines the number of sprays per dispensing action. Every spray will reduce the Actuation Count by one (620). For instance, if the Actuation Count is sets at three, the motor will be excited three times, causing the actuator to move downwards and back to its resting position three times resulting in three sprays of olfactory material into the atmosphere per dispensing action. On completion of the Actuation Routine, the timer measuring the time interval between dispensing actions will be reset and the routine return control to the Operation Routine.

The present invention provides advantages in that the status of the dispenser can be determined and the operational setting of the dispenser can be changed to suit different environmental conditions and multi-spray per dispensing action could be attained.

Although particular embodiments of the present invention have been described, those of skill in the art will appreciate that variations and modifications may be made without departing from the spirit and scope thereof as defined by the appended claims.

## Claims

1. An apparatus for dispensing olfactory stimulating material comprising:
a housing (10);
a deodorant source (70);
a power means (60);
control circuitry and display means (20), including a controller in operational contact with the assembly, wherein the controller can be configured by the user so as to set, for the dispenser, operational parameters including multiple sprays of olfactory material per dispensing action, the time during the day at which the dispenser will begin dispensing for the day, the time during the day at which the dispenser will stop dispensing for the day, and the frequency and/or intensity of dispensing;
user interface means (90, 91, 92, 93, 94, 95); and
dispensed material indicator means (20).

2. The apparatus as claimed in claim 1, whereby dispensed material computation is provided and olfactory material near empty status is indicated in display means (20).

3. The apparatus as claimed in claim 1 or 2, wherein an ambient condition sensor means (30) is provided to sense the state of ambient conditions and cause the controller to initiate preset actions when predetermined conditions are met.

4. The apparatus as claimed in claim 3, wherein said control circuitry incorporates a default setting, such that upon powering up the apparatus, default values for all the operating modes are loaded into the memory means of the apparatus.

5. The apparatus as claimed in claim 4, wherein the said apparatus incorporates an activating means (95) to set a fixed delay such that upon said means being activated, the apparatus will, after the fixed delay, wait for a change in ambient condition before performing some preset actions if predetermined conditions are met.
